Europäisches Patentamt

European Patent Office

Office européen des brevets

(11) Veröffentlichungsnummer: **0 220 453 B1**

# (12) EUROPÄISCHE PATENTSCHRIFT

(45) Veröffentlichungstag der Patentschrift: **15.04.92**

(21) Anmeldenummer: **86112691.0**

(22) Anmeldetag: **13.09.86**

Die Akte enthält technische Angaben, die nach dem Eingang der Anmeldung eingereicht wurden und die nicht in dieser Patentschrift enthalten sind.

(51) Int. Cl.5: **A61K 35/78**

(54) **Verwendung von Pflanzenpollenextrakten zur Herstellung von das Wachstum von Tumorzellen hemmenden pharmazeutischen Präparaten und Verfahren zu ihrer Herstellung.**

(30) Priorität: **20.09.85 CH 4089/85**

(43) Veröffentlichungstag der Anmeldung:
**06.05.87 Patentblatt 87/19**

(45) Bekanntmachung des Hinweises auf die Patenterteilung:
**15.04.92 Patentblatt 92/16**

(84) Benannte Vertragsstaaten:
**AT BE CH DE FR GB IT LI LU NL SE**

(56) Entgegenhaltungen:
**EP-A- 0 201 053**
**DE-A- 1 467 750**
**DE-B- 1 025 574**
**FR-A- 1 575 114**
**FR-A- 2 142 194**

(73) Patentinhaber: **Cernitin S.A.**

**CH-6911 Barbengo(CH)**

(72) Erfinder: **Lewenstein, Ari, Dr.**
**Via Marco Da Carona 5**
**CH-6900 Lugano(CH)**
Erfinder: **Fouad, Habib K., Dr.**
**6A, Albert Terrace**
**Edinburgh, EH10 5EA(GB)**

(74) Vertreter: **Blum, Rudolf Emil Ernst et al**
**c/o E. Blum & Co Patentanwälte Vorderberg 11**
**CH-8044 Zürich(CH)**

**Beschreibung**

HINTERGRUND DER ERFINDUNG:

In der Fachliteratur sind eine grosse Anzahl an Wirkstoffen beschrieben, die das Wachstum von Tumorzellen hemmen, bzw. Tumorzellen oder Leukämiezellen abtöten. Diese Wirkstoffe führen jedoch im allgemeinen auch bei gesunden Zellen zu einer gewissen Hemmung des Wachstums, wobei jedoch diese Wachstumshemmung sehr viel geringer ist, als die entsprechende Hemmung des Wachstums von Krebszellen.

Ziel der vorliegenden Erfindung war es, ein pharmazeutisches Präparat zu entwickeln, welches nur das Wachstum von Tumorzellen hemmt.

Ueberraschenderweise zeigte es sich, dass mit wässrigen Extraktionsmitteln aus Pflanzenpollen gewonnene Extrakte, bzw. an Aminosäuren und/oder Peptiden und/oder proteinartigen Substanzen angereicherte Fraktionen dieser Pollenextrakte das Wachstum von Tumorzellen hemmen und gleichzeitig die Stoffwechselvorgänge in gesunden Zellen stimulieren.

BESCHREIBUNG DES STANDES DER TECHNIK:

In der Literatur sind eine grosse Anzahl an Cytostatica beschrieben-und einige dieser Produkte wurden auch bereits zur chemotherapeutischen Behandlung von Tumoren eingesetzt. Es sei in diesem Zusammenhang auf die Veröffentlichung von Pirwitz "Grundlagen und Praxis chemischer Tumorbehandlungen", Berlin, Springer, 1954, verwiesen.

Ein wesentlicher Nachteil der meisten bisher bekannten zur Krebsbekämpfung eingesetzten Mittel besteht darin, dass sie nicht nur das Wachstum der Krebszellen hemmen, sondern auch gegenüber gesunden Zellen toxisch wirken. Es wurden bereits sehr viele Arbeiten durchgeführt, um Mittel zur Chemotherapie von Tumoren zu finden, welche diesen Nachteil nicht aufweisen. Beispielsweise wurden in der Veröffentlichung von B. Kellner et al. in "Naturwissenschaften", 1955, Seiten 582 und 583, cytostatische Mittel beschrieben, die auf bereits früher zur Chemotherapie von Tumoren eingesetzten Mittelnbasieren, wie zum Beispiel auf Stickstoff-Lost, wobei die Toxizität dieser Mittel durch eine chemische Bindung an Zucker, wie zum Beispiel an D-Mannit, vermindert wurde. In den Wurzeln bestimmter Berberitzengewächse wurden mitosehemmende Substanzen gefunden, die aus Glycosidoresten und einem aromatische Reste und cycloaliphatische Reste aufweisenden mehrkernigen Aglucon aufgebaut sind. Es sei in diesem Zusammenhang auf die Veröffentlichung von A. Stoll et al. in Helv. Chim. Acta Nr. 37, Seiten 1747 -1762, 1954, verwiesen.

Des weiteren sind Verfahren zur Herstellung von Extrakten aus Pflanzenpollen bekannt und es sei in diesem Zusammenhang beispielsweise auf in der deutschen Patentschrift Nr. 1 467 750 und der österreichischen Patentschrift Nr. 255 643 beschriebene Verfahren verwiesen. Nach diesen Verfahren gelingt es, Pollenextrakte zu gewinnen, die frei oder im wesentlichen frei von hochmolekularen Proteinen der Pollenhülle sind, die Allergien hervorrufen könnten. Bei den beschriebenen Verfahren wurden die Pollen mit einem wässrigen Extraktionsmittel extrahiert, anschliessend wurde mit einem Pilz der Gattung Mucor beimpft und in Anwesenheit dieses Pilzes wurden die hochmolekularen Proteine zu niedrigmolekularen Proteinen oder Aminosäuren abgebaut. Schliesslich wurde das wässrige Medium abgetrennt und getrocknet und der Pollenrückstand mit einem nicht wässrigen Medium weiter extrahiert und auch dieser zweite Extrakt gewonnen.

Die nach diesen bekannten Verfahren mit dem wässrigen Extraktionsmittel und dem nicht wässrigen Extraktionsmittel gewonnenen Pollenextrakte und auch Mischungen derselben, sind im Handel erhältlich und sie werden als Stärkungsmittel, zur Behandlung von Prostataleiden, zur Beschleunigung der Heilung von Wunden und Knochenbrüchen und als entzdüngshemmende Mittel eingesetzt.

In der nicht zum Stande der Technik gehörenden europäischen Patentanmeldung 86-106 017.6 (EP-A-201053) wird ferner ein Verfahren zur Herstellung eines pharmazeutischen Präparates zur prophylaktischen Behandlung von Allergien beschrieben, das als Wirkstoff einen mit wässrigen Extraktionsmitteln oder mit nicht wässrigen Extraktionsmitteln gewonnenen Pollenextrakt enthält.

Ueberraschenderweise zeigte es sich jetzt, dass ein mit einem wässrigen Extraktionsmittel gewonnener Extrakt aus Pflanzenpollen das Wachstum von Tumorzellen hemmt.

BESCHREIBUNG DER ERFINDUNG

Ein Gegenstand der vorliegenden Erfindung ist die Verwendung von Pflanzenpollenextrakten zur Herstellung von pharmazeutischen Präparaten zur Hemmung des Tumorwachstums, wobei man einen mit einem wässrigen Extraktionsmittel aus Pflanzenpollen gewonnenen Extrakt verwendet, der höchstens 5 Gew.-% an hochmolekularen Proteinen enthält oder frei von diesen ist.

Bevorzugte Extrakte, die erfindungsgemäss zur Herstellung der pharmazeutischen Präparate verwendet werden, sind entsprechende getrocknete Extrakte, die einen Wassergehalt von unter 12%, bezogen auf das Gesamtgewicht des Extraktes, aufweisen und die mindestens 70 Gew.-% an Kohlehydraten, 5 - 12 Gew.-% an Aminosäuren und/oder Peptiden, sowie wasserlösliche Vitamine der B-Gruppe enthalten.

Zur Testung der hemmenden Wirkung, welche die Pflanzenpollenextrakte auf Krebszellen ausüben, wurde eine grosse Anzahl an Zellen herangezogen, wie zum Beispiel menschliche Kehlkopfkrebszellen, menschliche Leberkrebszellen, Blasenkrebszellen, Prostatakrebszellen, Hodenkrebszellen und menschliche Brustkrebszellen, und es wurden auch Leukämiezellen getestet. Die Ueberlebensrate der Zellen wurde durch eine Zählung der Zellen am Beginn der Kultur und nach einer bestimmten Züchtungsphase festgestellt.

Bei den Versuchen zu Vergleichszwecken wurden die fraglichen Zellkulturen ohne Zugabe der mit wässrigen Extraktionsmitteln gewonnenen Pflanzenpollenextrakte gezüchtet.

Bei den Versuchen zur Bestimmung der Hemmung des Wachstums der Tumorzellen wurde nach einer Anzuchtphase der mit den wässrigen Extraktionsmitteln gewonnene Pflanzenpollenextrakt zugegeben.

In allen Fällen wurden vor den jeweiligen Zählungen Färbungen mit Trypanblau vorgenommen, bei denen die abgestorbenen Zellen gefärbt wurden.

Ueberraschenderweise zeigte es sich, dass bei einigen Arten der getesteten Krebszellen sofort nach der Zugabe der erfindungsgemäss zu verwendenden Pflanzenpollenextrakte ein geringfügiges Ansteigen der Zellzahlen der Krebszellen festgestellt wurde. In diesen Fällen folgte jedoch nach einer fortgesetzten Behandlung von zwei oder mehr Tagen dann eine Abnahme der lebenden Krebszellen in den Kulturen. Bei einigen der getesteten Krebszellen erfolgte gleich nach der Zugabe der erfindungsgemäss zu verwendenden Pflanzenpollenextrakte eine Abnahme der lebenden Krebszellen, im Vergleich zu den Vergleichsversuchen, die ohne Zugabe dieser Extrakte weitergezüchtet wurden.

Besonders stark erwies sich die wachstumshemmende Wirkung der erfindungsgemäss zu verwendenden Pflanzenpollenextrakte auf das Wachstum von Leukämiezellen. In diesem Fall wurde in den Kulturen sofort nach der Zugabe der erfindungsgemäss zu verwendenden Pflanzenpollenextrakte das Wachstum der Leukämiezellen gehemmt und ab dem zweiten Tag der Behandlung konnten überhaupt keine lebenden Leukämiezellen mehr nachgewiesen werden.

Bei den übrigen getesteten Zellen war eine deutliche Hemmung des Zellwachstums bei Zugabe der erfindungsgemäss zu verwendenden Pflanzenpollenextrakte feststellbar. Die Ergebenisse waren für die übrigen getesteten Krebsarten ziemlich ähnlich, d.h. es konnten überall deutliche Wachstumshemmungen festgestellt werden, jedoch ein sofortiges Absterben aller lebenden Krebszellen, bereits am zweiten Tage nach der Behandlung, konnte nur bei den Leukämiezellen festgestellt werden.

Es konnte ferner gezeigt werden, dass entsprechende Zellkulturen, die gesunde menschliche Zellen enthielten, beispielsweise gesunde menschliche Prostatazellen oder gesunde menschliche Brustzellen, durch die Zugabe der erfindungsgemäss zu verwendenden mit wässrigen Extraktionsmitteln gewonnenen Pollenextrakte in ihrem Wachstum nicht gehemmt wurden und dass sogar ein erhöhtes Wachstum, im Vergleich zu entsprechenden Vergleichsproben ohne Zugabe der Pollenextrakte, festgestellt werden konnte.

Aus diesen Testergebnissen sieht man also, dass die mit wässrigen Extraktionsmitteln gewonnenen Pflanzenpollenextrakte Wirkstoffe enthalten, welche spezifisch das Wachstum von Krebszellen hemmen, und dass diese Pollenextrakte gegenüber Leukämiezellen besonders wirksam sind, indem sie in kurzer Zeit zu einem Absterben sämtlicher Leukämiezellen führen.

Gemäss einer bevorzugten Ausführungsart der vorliegenden Erfindung wird zur Herstellung des pharmazeutisches Präparates ein Pollenextrakt verwendet, der durch Extraktion getrockneter Pflanzenpollen mit einem Extraktionsmittel, enthaltend Wasser und ein wassermisch bares organisches Lösungsmittel und durch den Abbau allenfalls vorhandener hochmolekularer Proteine durch Einwirkung von Mikroorganismen erhalten wurde, wobei der Abbau der hochmolekularen Proteine unter Bildung von niedrigmolekularen Proteinen oder Aminosäuren vollständig oder bis zu einem Maximalgehalt von 5 Gew.-% an hochmolekularen Proteinen, bezogen auf das Trockengewicht, durchgeführt wurde und wobei das von den Pollenrückständen abgetrennte Wasser enthaltene Extraktionsmedium nach der Abdampfung unter Vakuum das zu verwendende Wirkstoffgemisch in Form eines gelblich-weissen Produktes liefert.

Das nach diesem Extraktionsverfahren erhaltene Wirkstoffgemisch in Form eines gelblich-weissen Produktes weist im allgemeinen einen Wassergehalt von unter 12% auf und es enthält, bezogen auf das Gesamtgewicht, mindestens 70% an Kohlehydraten und 5 - 12% an Aminosäuren, Peptiden oder proteinartigen Substanzen, sowie ferner wasserlösliche Vitamine der Gruppe B. Der Aschegehalt dieser getrockenten Präparate ist relativ hoch und deutet auf einen grossen Gehalt an Mineralstoffen hin.

Die chemische Untersuchung typischer so gewonnener trockener Präparate ergab die folgenden Werte:

| Bestandteil | Menge in Gew.-% |
|---|---|
| Wasser | 5 - 10 |
| Stickstoff | 2,5 |
| Asche | 4,5 |
| Aminosäuren, Proteine und proteinartige Substanzen | 6 - 9 |
| freies Phosphat | 0,8 - 1,4 |
| Kohlehydrate | 78 |
| Zuckerbestandteile | 5 - 10 |

Das Trockenprodukt enthielt ferner 7,5 $\mu$g an Vitamin B$^2$ pro Gramm und 60 $\mu$g an Vitamin B$^3$ pro Gramm.

Die Untersuchung der mineralischen Bestandteile der mit den wässrigen Extraktionsmitteln nach dem beschriebenen Verfahren gewonnenen Extrakte ergab die folgenden Werte:

| Bestandteil | Menge in ppm |
|---|---|
| Ca | 56 |
| P | 58 |
| Mg | 11 |
| Na | 250 |
| Fe | 18 |
| Zn | 2,3 |
| Cu | 5 |
| Al | 12 |

Auch die Zuckerbestandteile der Extrakte wurden näher untersucht, und zwar konnte man darin Arabinose, Galaktose, Mannose und Glucose feststellen, jeweils in Mengen von Spuren bis 4 % oder mehr.

Die Aktivität der mit den wässrigen Extraktionsmitteln gewonnenen Pollenextrakte, die Stoffwechselvorgänge in gesunden Zellen zu stimulieren, wurden mit Hilfe von in vitro Versuchen bestimmt. Hiezu wurde der zyklische Adenosin-Monophosphat-Test, abgekürzt als c-AMP - Test, durchgeführt. Zu diesem Test benützte man hochgereinigte Leydigzellen in einer Konzentration von 10$^5$ Zellen/ml. Der c-AMP - Test stellt ein Defizitmodell dar, in dem man vorgealterte Zellen, in diesem Fall 24 Stunden bei 6° C, mit einem Peptidhormon, dem hcg, stimuliert und radioimmunologisch die c-AMP-Produktion überprüft. Sollte nun durch die Zuführen des zu untersuchenden Substrates die c-AMP-Produktion stimuliert werden, so muss dieses Substrat in sich Substanzen tragen, die durch den Alterungsprozess entstandenen Defizite aufheben und somit eine Stimulation des Zellstoffwechsels bewirken.

Es zeigte sich, dass die erfindungsgemäss verwendeten Pollenextrakte eine deutliche Kumulierung der c-AMP-Produktion hervorriefen, und zwar im Vergleich zu entsprechenden Tests, wo kein derartiger Pollenextrakt zugesetzt wurde.

Der fragliche Test wurde auch anstatt an hochgereinigten Leydigzellen an entsprechenden Prostatazellen vorgenommen und in diesem Falle konnten analoge Ergebnisse erzielt werden.

Die wässrigen Extraktionsmittel, die zur Herstellung der Pollenextrakte eingesetzt werden, sind vorzugsweise Mischungen aus Wasser mit einem gewichtsmässig geringeren Anteil an einem völlig wassermischbaren organischen Lösungsmittel. Beispiele für derartige organische Lösungsmittel sind niedere Alkohole oder niedere Ketone, wie zum Beispiel Aethylalkohol oder Aceton. Das verwendete wässrige Extraktionsmittel weist im allgemeinen einen Gehalt von 2 - 30 % an dem mit Wasser mischbaren organischen Lösungsmittel auf, wobei der Rest auf 100 Gew.-% aus Wasser besteht, vorzugsweise aus destilliertem Wasser.

Als Beispiele für gut geeignete wässrige Extraktionsmittel sei eine 20 %-ige wässrige Lösung von Aethylalkohol oder eine 5 %-ige wässrige Lösung von Aceton genannt.

Nach dieser Vorextraktionsbehandlung belässt man die Pollen in Anwesenheit von Mikroorganismen in dem wässrigen Extraktionsmedium unter Rühren während weiterer 36 bis 60 Stunden bei einer Temperatur

von 15 - 35º C. Während dieser Zeit erfolgt die Fermentierung der äusseren Pollenhüllen. Die Mikroorganismen bauen die hochmolekularen Proteine zu niedrigmolekularen Proteinen oder freien Aminosäuren ab.

Als Allergen wirkende Bestandteile, die in den als Ausgangsmaterial eingesetzten Pollen enthalten sein können, sind hochmolekulare Proteine. Diese werden durch die genannte Einwirkung der Mikroorganismen zumindestens so weit abgebaut, dass das nach der Fermentation erhaltene Produkt keine allergene Wirkung zeigt.

Als Mikroorganismen werden für die Durchführung der Fermentation mit Vorteil Pilze verwendet. Pilze, die für diese Fermentation gut geeignet sind, sind solche der Gattung Mucor. Als Beispiel für derartige Pilze, welche den Abbau der hochmolekularen Proteine bewirken, seien genannt: Mucor glomerata; Mucor luteus; Mucor mucedo; Mucor flavus, Mucor corticolus, Mucor brunneus, Mucor hiemalis oder Mucor alpinus.

Nach dieser Fermentationsbehandlung erfolgt ein Filtrieren. Das Filtrat stellt eine klare Lösung dar. Aus diesem Filtrat wird das wässrige Medium unter Vakuum abgedampft, wobei man einen entsprechenden Pollenextrakt in Form eines gelblich-weissen Produktes erhält. Dieses Wirkstoffgemisch, das im allgemeinen die weiter vorne angegebene Zusammensetzung an organischen Substanzen, Vitaminen und mineralischen Bestandteilen enthält, wird erfindungsgemäss zur Herstellung der tumor hemmenden pharmazeutischen Präparate nach Anspruch verwendet. Bezüglich der Auswahl der Pflanzenpollen und bezüglich weiterer Einzelheiten des Verfahrens zur Herstellung der mit wässrigen Extraktionsmitteln gewonnenen Pollenextrakte sei auf dasjenige verwiesen, was in der deutschen Patentschrift Nr. 1 467 750 und der österreichischen Patentschrift Nr. 255 643 bereits erläutert wird. Des weiteren wird auch in der nicht zum Stande der Technik gehörenden europäischen Patentanmeldung 86-106 017.6 (EP-A-201053) das Verfahren zur Herstellung von Pollenextrakten unter Verwendung von wässrigen Extraktionsmitteln näher erläutert und dort wird auch ein Verfahren zur Herstellung von Pollenextrakten unter Verwendung von nicht wässrigen Extraktionzmitteln beschrieben. Es sei jedoch darauf hingewiesen, dass nur die mit den wässrigen Extraktionsmitteln gewonnenen Pollenextrakte die Wirkstoffe enthalten, welche das Wachstum der Tumorzellen hemmen, während eine derartige Aktivität bei den mit den nicht wässrigen Extraktionsmitteln erhaltenen Pollenextraktennicht festgestellt werden konnte.

Aus den mit den wässrigen Extraktionsmitteln hergestellten Pollenextrakten, die in Form des gelblich-weissen Produktes erhalten wurden, wurden dann Fraktionen hergestellt.

Bisher wurden aus den mit wässrigen Extraktionsmitteln hergestellten Pollenextrakten noch keine Fraktionen isoliert und als Wirkstoff eingesetzt. Bei den entsprechenden Wirkstoff-Fraktionen handelt es sich also um neue, bisher noch nicht beschriebene Produkte.

Gemäss einer Ausführungsart der vorliegenden Erfindung wird als Wirkstoff eine aus dem gelblichweissen Produkt gewonnene Fraktion verwendet, wobei diese Fraktion ein Molekulargewicht unter 750 aufweist. Eine bevorzugte als Wirkstoff eingesetzte Fraktion weist ein Molekulargewicht auf, das im Bereich von 650 - 750 liegt.

Ein weiterer Gegenstand der vorliegenden Erfindung ist ein Verfahren zur Isolierung von neuen Wirkstoff-Fraktionen von Pflanzenpollenextrakten, bei dem man die getrockneten Pflanzenpollen mit einem Extraktionsmittel, enthaltend Wasser und ein wassermischbares organisches Lösungsmittel, extrahiert, allenfalls vorhandene hochmolekulare Proteine vollständig oder bis zu einem Maximalgehalt von 5 Gew.-% an hochmolekularen Proteinen durch Einwirkung von Mikroorganismen zu niedrigmolekularen Proteinen oder Aminosäuren abbaut, das wässrige Medium von den Pollenrückständen abtrennt und unter Vakuum eindampft, wobei das Wirkstoffgemisch in Form eines gelblichweissen Produktes zurückbleibt, und dieses Verfahren dadurch gekennzeichnet ist, dass man aus diesem Wirkstoffgemisch durch Gelelektrophorese eine neue Wirkstoff-Fraktion isoliert, deren Molekulargewicht unter 750 liegt, wobei vorzugsweise diejenige Fraktion isoliert wird, deren Molekulargewicht im Bereich von 650 - 750 liegt.

Weitere bevorzugte Fraktionen, die man erfindungsgemäß als Wirkstoff verwendet, sind aus dem gelblich-weissen Produkt isolierte, an Aminosäuren und/oder Peptiden angereicherte Fraktionen, wobei diese Fraktionen erhalten wurden, indem man das gelblich-weisse Produkt in Wasser löste, ein mit Wasser mischbares organisches Lösungsmittel und gegebenenfalls eine organische Säure zur Ausfällung der Aminosäuren, Proteine und/oder proteinartigen Substanzen zusetzte und den ausgefällten isolierten Wirkstoff nach dem Trocknen als Wirkstoff-Fraktion einsetzte.

Gemäss einer anderen Ausführungsart der Erfindung werden diejenigen, an Aminosäuren und/oder Peptiden und/oder proteinartigen Substanzen angereicherten Fraktionen verwendet, die durch eine säulenchromatographische Trennung des gelblich-weissen Produktes und Isolierung, Vereinigung und Abdampfung derjenigen Fraktionen erhalten wurden, in denen durch den Ninhydrin-Test die Anwesenheit von Aminosäuren und/oder Peptiden und/oder proteinartigen Substanzen nachweisbar ist. Herstellung von erfindundsgemäß verwendbaren, am Aminosäuren und/oder Peptiden und/oder proteinartigen Substanzen angereicherten Fraktionen von Pflanzenpollenextrakten, werden also die getrockneten Pflanzenpollen mit einem Extraktionsmittel, enthaltend Wasser und ein wassermischbares organisches Lösungsmittel, extrahiert, anschliessend werden allenfalls vorhandene hochmolekulare Proteine vollständig oder bis zu einem Maximalgehalt von 5 Gew.-%, bezogen auf das Trockengewicht, durch Einwirkung von Mikroorganismen zu niedrigmolekularen Proteinen oder Aminosäuren abbgeaut, das wässrige Medium von dem Pollenrückstand abgetrennt und unter Vakuum eingedampft, wobei ein Wirkstoffgemisch in Form eines gelblich-weissen Produktes zurückbleibt und aus diesem Wirkstoffgemisch wird dann eine an Aminosäuren und/oder Peptiden und/oder proteinartigen Substanzen angereicherte Fraktion herstellt, indem man entweder

a) das gelblich-weisse Produkt in Wasser löst, ein mit Wasser mischbares organisches Lösungsmittel, beispielsweise einen niederen Alkohol oder ein niederes Keton und gegebenenfalls eine organische Säure zusetzt und dadurch Aminosäuren, Peptide und/oder proteinartige Substanzen ausfällt und den Niederschlag isoliert und trocknet oder indem man

b) aus dem gelblich-weissen Produkt eine an Aminosäuren und/oder Peptiden und/oder proteinartigen Substanzen angereicherte Fraktion herstellt, indem man das gelblich-weisse Produkt einer säulenchromatographischen Trennung unterwirft, beispielsweise unter Verwendung einer mit Sephadex ® gefüllten Säule, und diejenigen Fraktionen auffängt, in denen durch den Ninhydrintest die Anwesenheit von Aminosäuren und/oder Peptiden und/oder proteinartigen Substanzen nachgewiesen werden kann und aus diesen Fraktionen das Lösungsmittel abdampft.

Bei der Herstellung der Wirkstoff-Fraktion zur erfindungsgemäßen Verwendung nach der Ausführungsform (a) des oben beschriebenen Verfahrens kann beispielsweise das gelblich-weisse Produkt in Wasser gelöst werden und anschliessend setzt man zur Ausfällung der gewünschten Fraktion das wassermischbare organische Lösungsmittel, vorzugsweise einen niederen Alkohol oder ein niederes Keton und allenfalls eine aliphatische oder aromatische Carbonsäure zu, beispielsweise Benzoesäure. Gemäss einer speziellen Ausführungsart erfolgt die Ausfällung durch Zugabe von Aceton und Benzoesäure.

Die neuen aus den Pflanzenpollenextrakten isolierten Fraktionen werden derzeit bezüglich ihrer pharmakologischen Aktivität noch näher untersucht.

Die Erfindung sei anhand der nachfolgenden Beispiele näher erläutert. Sämtliche Tests, die an Krebszellen durchgeführt wurden, wurden mit dem nach dem Verfahren gemäss Beispiel 1 hergestellten Pflanzenpollenextrakt durchgeführt oder mit Pflanzenpollenextrakten, die nach ähnlichen Verfahren erhalten wurden. Die an den Proteinen angereicherten Fraktionen, die nach dem Verfahren gemäss den Beispielen 2 und 3 hergestellt wurden, sind neue Produkte, die jedoch bezüglich ihrer krebshemmenden Aktivitäten noch nicht entsprechend getestet werden konnten.

## Beispiel 1

120 kg Pollen von Roggen mit der lateinischen Bezeichnung Secale cereale werden mit 600 kg einer 20 molprozentigen Lösung von Aethylalkohol in Wasser behandelt. Man rührt die Pollen während 48 Stunden bei einer Temperatur von 30 - 32º C. Nach dieser Vorbehandlung setzt man Mucor alpinus zu und rührt bei 30º C während 20 - 30 Stunden.

Das wässrige Medium wird von den Pollen abfiltriert. Es stellt eine klare Lösung dar. Diese klare Lösung wurde steril filtriert und aus ihr wurde dann das Lösungsmittel unter Vakuum abgedampft. Man erhielt dabei einen getrockneten Pflanzenpollenextrakt. Dieser stellte ein geblich-weisses Pulver dar. Dieses gelblich-weisse Pulver wurde erfindungsgemäss zur Herstellung der pharmazeutischen Präparate verwendet, welche das Wachstum von Tumorzellen hemmen und die Stoffwechselvorgänge in gesunden Zellen stimulieren.

## Beispiel 2

Das nach dem Verfahren gemäss Beispiel 1 gewonnene Produkt wurde in Wasser gelöst, bis eine vollständige Lösung erreicht war. Anschliessend wurden Aceton und Benzoesäure zugesetzt. Das Aceton wurde tropfenweise zugegeben, wobei sich ein Niederschlag bildete und die Zugabe an Aceton erfolgte so lang, bis kein weiteres Material mehr ausfiel. Der gebildete Niederschlag wurde aus dem flüssigen Medium entfernt. beispielsweise durch Filtrieren oder durch Absaugen des Lösungsmittels. Anschliessend wurde getrocknet.

Das so erhaltene Trockenprodukt wurde in einer Menge von etwa 60 Gew.-%, bezogen auf die 100 Gew.-% des in diesem Beispiel verwendeten Ursprungsextraktes gemäss Beispiel 1 isoliert.

Die Mutterlauge, die verworfen wurde, war völlig frei von proteinartigen Materialien oder Peptiden oder im wesentlichen frei von diesen Produkten. Man sieht daraus dass in dem erhaltenen getrockneten Niederschlag eine wesentliche Anreicherung an Peptiden und proteinartigen Komponenten, verglichen mit dem urpsrünglichen Extraxt, stattgefunden hatte.

Beispiel 3

Die nach dem Verfahren gemäss Beispiel 2 erhaltene an Peptiden und proteinhaltigen Materialien, sowie Aminosäuren angereicherte Fraktion wurde der Mikrogradienten-gelelektrophorese unterworfen. Der Peak mit einem Molekulargewicht von etwa 700 Da wurde isoliert, die Menge an dem darin enthaltenen Peptid betrug 1 Gew.-%, bezogen auf den gemäss Beispiel 1 hergestellten getrockneten Extrakt.

Beispiel 4

In diesem Beispiel wurde die wachstumshemmende Wirkung von erfindungsgemäss zu verwendenden, mit wässrigen Extraktionsmitteln gewonnenen Pollenextrakten auf Leukämiezellen getestet.

Die Zellkulturen enthielten am Beginn des Tests jeweils $4 \times 10^4$ Leukämiezellen pro ml. Bei den Zellkulturen zu Vergleichszwecken, bei denenkein mit wässrigen Extraktionsmitteln gewonnener Pollenextrakt zugesetzt wurde, war die Anzahl der lebenden Leukämiezellen bis zum vierten Tag der Züchtung in den entsprechenden Kulturen auf $5,2 \times 10^5$ Zellen pro ml angestiegen.

Bei denjenigen Kulturen, bei denen der Pollenextrakt in einer Menge von 1 mg/ml Kultur zugesetzt wurde, war am Ende des ersten Tages der Behandlung die Anzahl der lebenden Leukämiezellen von $4 \times 10^4$ lebenden Zellen auf $4 \times 10^3$ lebenden Zellen gesunken, und ab dem zweiten Tage konnten überhaupt keine lebenden Zellen mehr festgestellt werden.

Bei einer weiteren Serie von Zellkulturen wurde zu den lebenden Leukämiezellen der Pollenextrakt in einer Menge von 4 mg/ml Zellkultur zugesetzt. In diesem Falle war schon am Ende des ersten Behandlungstages die Anzahl der lebenden Leukämiezellen von $4,0 \times 10^4$ Zellen pro ml auf $2 \times 10^3$ Zellen pro ml gesunken, und am zweiten Tag konnten ebenfalls überhaupt keine lebenden Zellen mehr festgestellt werden.

Die entsprechenden Ergebnisse sind in der nachfolgenden Tabelle zusammengestellt.

| Tage nach Beginn der Behandlung | Anzahl der lebenden Leukämiezellen pro ml nach Zugabe des Pollenextraktes in einer Menge von | | |
|---|---|---|---|
| | 0 | 1 mg/ml | 4 mg/ml |
| 1 | $4,0 \times 10^4$ | $4 \times 10^3$ | $2 \times 10^3$ |
| 2 | $6,0 \times 10^4$ | 0 | 0 |
| 3 | $2,2 \times 10^5$ | 0 | 0 |
| 4 | $5,2 \times 10^5$ | 0 | 0 |

Unter den gleichen Bedingungen, wie die Leukämiezellen wurden normale menschliche weisse Blutkörperchen gezüchtet. Auch in diesem Falle wurde bei den Zellkulturen zu Vergleichszwecken ohne Zugabe von Pollenextrakt drei Tage weitergezüchtet. Eine Serie von Kulturen wurde unter Zugabe von 1 mg/ml Kultur des Pollenextraktes weitergezüchtet und eine weitere Serie von Kulturen unter Zugabe von 4 mg/ml des Pollenextraktes. In sämtlichen Kulturen und auch in der Kultur zu Vergleichszwecken konnte während der drei Tage der Züchtung eine geringfügige Abnahme der Zellzahlen festgestellt werden. Es waren aber keine drastischen Unterschiede zwischen den behandelten Kulturen und den nicht behandelten Kulturen festzustellen.

Aus diesen Ergebnissen sieht man, dass die erfindungsgemäss zu verwendenden, mit wässrigen Extraktionsmitteln gewonnenen Pollenextrakte selektiv die Leukämiezellen abtöten, jedoch das Wachstum der gesunden weissen Blutkörperchen nicht behindern.

Beispiel 5

In diesem Beispiel wurden Zellkulturen von menschlichen Prostatakrebszellen und menschlichen Hodenkrebszellen untersucht. Auch in diesem Falle wurde bei den Zellkulturen zu Vergleichszwecken ohne Zugabe des mit den wässrigen Extraktionsmitteln gewonnenen Pollenextraktes weitergezüchtet, während eine Serie von Zellkulturen unter Zugabe von 1 mg/ml des Pollenextraktes und eine weitere Serie unter Zugabe von 4 mg/ml der Kultur des Pollenextraktes weitergezüchtet wurde.

7

Bei den entsprechenden Kulturen der Prostatakrebszellen war in der Gruppe zu Vergleichszwecken ein deutlicher Anstieg der Anzahl der lebenden Krebszellen von ursprünglich 4,4 x $10^4$ Zellen pro ml auf 1,2 x $10^5$ Zellen pro ml am vierten Tag der Züchtung festzustellen.

Bei denjenigen Kulturen, wo unter Zugabe von 1 mg/ml des Pollenextraktes weitergezüchtet wurde, war der Anstieg der lebenden Krebszellen deutlich geringer, d.h. die Zellzahl war von 4,4 x $10^4$ Zellen pro ml am vierten Tag auf 7,6 x $10^4$ Zellen pro ml angestiegen.

Bei denjenigen Kulturen, bei denen 4 mg/ml des mit wässrigen Extraktionsmitteln gewonnenen Pollenextraktes zugesetzt wurde, trat ein deutliches Absinken der Zellzahlen auf, nämlich die Anzahl der lebenden Prostatakrebszellen war von 4,4 x $10^4$ Zellen pro ml vor Beginn der Behandlung auf 6,0 x $10^3$ lebende Zellen am vierten Tag nach der Behandlung gesunken. Die entsprechenden Ergebnisse sind in der nachfolgenden Tabelle zusammengestellt.

Zellkulturen von menschlichen Prostatakrebszellen

| Tage nach Beginn der Behandlung | Anzahl der lebenden Krebszellen pro ml nach Zugabe des Pollenextraktes in einer Menge von | | |
|---|---|---|---|
| | 0 | 1 mg/ml | 4 mg/ml |
| 1 | 4,4 x $10^4$ | 3,8 x $10^4$ | 4,2 x $10^4$ |
| 2 | 6,0 x $10^4$ | 3,2 x $10^4$ | 3,6 x $10^4$ |
| 3 | 1,0 x $10^5$ | 4,4 x $10^4$ | 1,2 x $10^4$ |
| 4 | 1,2 x $10^5$ | 7,6 x $10^4$ | 6,0 x $10^3$ |

Bei den Zellkulturen der menschlichen Hodenkrebszellen trat bei der Gruppe zu Vergleichszwecken während der Züchtung bis zum vierten Tage eine geringfügige Abnahme der lebenden Zellen auf. Bei denjenigen Kulturen. bei denen der Pollenextrakt in einer Menge von 1 mg/ml zugesetzt wurde, war am vierten Tag der Züchtung die Zahl der lebenden Krebszellen wesentlich stärker gesunken als in der Gruppe zu Vergleichszwecken und die höhere Dosis von 4 mg Pollenextrakt pro ml führte zu einer noch wesentlich stärkeren Verminderung der Anzahl der lebenden Zellen.

Aehnliche Ergebnisse wie bei den Zellkulturen der menschlichen Prostatakrebszellen wurden bei Zellkulturen erhalten, die menschliche Kehlkopfkrebszellen, menschliche Leberkrebszellen, menschliche Blasenkrebszellen und menschliche Brustkrebszellen enthielten.

**Patentansprüche**

1. Verwendung von Pflanzenpollenextrakten zur Herstellung von pharmazeutischen Präparaten zur Hemmung des Tumorwachstums, wobei man einen mit einem wässrigen Extraktionsmittel aus Pflanzenpollen gewonnenen Extrakt verwendet, der höchstens 5 Gew.-% an hochmolekularen Proteinen enthält oder frei von diesen ist.

2. Verwendung nach Anspruch 1, wobei der Extrakt zur Herstellung eines pharmazeutischen Präparates verwendet wird, welches das Wachstum von Krebszellen nach dem Ablauf einer Startphase hemmt, bzw. das Wachstum von Leukämiezellen gleich nach Beginn nach der Behandlung hemmt und diese gegebenenfalls vollständig abtötet.

3. Verwendung nach Anspruch 1 oder 2, wobei man einen getrockneten Extrakt verwendet, der einen Wassergehalt von unter 12%, bezogen auf das Gesamtgewicht des Extraktes, aufweist und der mindestens 70 Gew.-% an Kohlehydraten, 5 - 12 Gew.-% an Aminosäuren und/oder Peptiden, sowie wasserlösliche Vitamine der B-Gruppe enthält.

4. Verwendung nach Anspruch 1 oder 2, wobei man zur Herstellung des pharmazeutischen Präparates eine an Aminosäuren und/oder Peptiden und/oder proteinartigen Substanzen angereicherte Fraktion des Pollenextraktes verwendet.

5. Verwendung nach einem der Ansprüche 1 - 4, indem man zur Herstellung des pharmazeutischen Präparates einen Pollenextrakt verwendet, der durch Extraktion getrockneter Pflanzenpollen mit einem Extraktionsmittel, enthaltend Wasser und ein wassermischbares organisches Lösungsmittel und den Abbau allenfalls vorhandener hochmolekularer Proteine durch Einwirkung von Mikroorganismen erhalten wurde, wobei der Abbau der hochmolekularen Proteine unter Bildung von niedrigmolekularen Proteinen oder Aminosäuren vollständig oder bis zu einem Maximalgehalt von 5 Gew.-% an hochmolekularen Proteinen, bezogen auf das Trockengewicht, durchgeführt wurde und wobei das von den Pollenrückständen abgetrennte Wasser enthaltende Extraktionsmedium nach der Abdampfung unter Vakuum das zu verwendende Wirkstoffgemisch in Form eines gelblich-weissen Produktes liefert.

6. Verwendung nach Anspruch 5, dadurch gekennzeichnet, dass man als Wirkstoff eine aus dem gelblich-weissen Produkt isolierte, an Aminosäuren und/oder Peptiden angereicherte Fraktion verwendet, wobei diese Fraktion hergestellt wurde, indem man das gelblichweisse Produkt in Wasser löste, ein mit Wasser mischbares organisches Lösungsmittel und gegebenenfalls eine organische Säure zur Ausfällung der Aminosäuren, Proteine und/oder proteinartigen Substanzen zusetzte und den ausgefällten isolierten Niederschlag nach dem Trocknen als Wirkstoff-Fraktion einsetzt.

7. Verwendung nach Anspruch 5, dadurch gekennzeichnet, dass man als Wirkstoff eine aus dem gelblich-weissen Produkt gewonnene, an Aminosäuren und/oder Peptiden und/oder proteinartigen Substanzen angereicherte Fraktion verwendet, wobei die Fraktion durch eine säulenchromatographische Trennung des gelblich-weissen Produktes und Isolierung, Vereinigung und Abdampfung derjenigen Fraktionen erhalten wurde, in denen durch den Ninhydrin-Test die Anwesenheit von Aminosäuren und/oder Peptiden und/oder proteinartigen Substanzen nachweisbar ist.

8. Verwendung nach Anspruch 5, dadurch gekennzeichnet, dass man als Wirkstoff eine aus dem gelblich-weissen Produkt gewonnene Fraktion verwendet, wobei das Molekulargewicht der Fraktion unter 750, vorzugsweise im Bereich von 650 - 750 liegt.

9. Verfahren zur Isolierung von neuen Wirkstoff-Fraktionen von Pflanzenpollenextrakten, bei dem man die getrockneten Pflanzenpollen mit einem Extraktionsmittel, enthaltend Wasser und ein wassermischbares organisches Lösungmittel, extrahiert, allenfalls vorhandene hochmolekulare Proteine vollständig oder bis zu einem Maximalgehalt von 5 Gew.-% an hochmolekularen Proteinen durch Einwirkung von Mikroorganismen zu niedrigmolekularen Proteinen oder Aminosäuren abbaut, das wässrige Medium von den Pollenrückständen abtrennt und unter Vakuum eindampft, wobei das Wirkstoffgemisch in Form eines gelblichweissen Produktes zurückbleibt, dadurch gekennzeichnet, dass man aus diesem Wirkstoffgemisch durch Gelelektrophorese eine neue Wirkstoff-Fraktion isoliert, deren Molekulargewicht unter 750 liegt, wobei vorzugsweise diejenige Fraktion isoliert wird, deren Molekulargewicht im Bereich von 650 - 750 liegt.

10. Aus den Pflanzenpollenextrakten isolierte Wirkstoff-Fraktionen, dadurch gekennzeichnet, dass diese Wirkstoff-Fraktionen nach dem Verfahren gemäss Anspruch 9 isoliert werden.

**Claims**

1. Use of plant pollen extracts for the manufacture of pharmaceutical preparations for the inhibition of tumour growth, whereby an extract obtained from plant pollen by an aqueous extraction means is used which contains at most 5% by weight high-molecular proteins or is free of these.

2. Use according to claim 1, whereby the extract for the manufacture of a pharmaceutical preparation is used which inhibits the growth of cancer cells after the course of a starting phase, or inhibits the growth of leukaemia cells straight after the beginning of the treatment and if necessary kills them completely.

3. Use according to claim 1 or 2, whereby a dried extract is used which has a water content of below 12%, relative to the total weight of the extract, and which contains at least 70% by weight carbohydrates, 5-12% by weight amino acids and/or peptides, as well as water-soluble vitamins of the B-group.

4. Use according to claim 1 or 2, whereby for the manufacture of the pharmaceutical preparation a fraction of the pollen extract is used which is enriched with amino acids and/or peptides and/or protein-like substances.

**5.** Use according to one of claims 1-4, in that for the manufacture of the pharmaceutical preparation a pollen extract is used which was obtained through extraction of dried plant pollens by an extraction means, containing water and a water-mixable organic solvent, and through the break-down of potential high-molecular proteins through the action of micro-organisms, whereby the break-down of the high-molecular proteins was carried out with the formation of low-molecular proteins or amino acids completely or up to a maximum content of 5% by weight high-molecular proteins relative to the dry weight, and whereby the extraction medium, containing water separated from the pollen residues, after the evaporation by vacuum, supplies the active-substance mixture to be used in the form of a yellowish-white product.

**6.** Use according to claim 5, characterized in that as active substance a fraction is used which is isolated from the yellowish-white product and enriched with amino acids and/or peptides, whereby this fraction was manufactured in that the yellowish-white product was dissolved in water, an organic solvent mixable with water was added and if necessary an organic acid for the precipitation of the amino acids, proteins and/or protein-like substances was added, and the precipitated isolated deposit is used as active-substance fraction after the drying.

**7.** Use according to claim 5, characterized in that a fraction obtained from the yellowish-white product and enriched with amino acids and/or peptides and/or protein-like substances is used as active substance, whereby the fraction was obtained by a column-chromatographic separation of the yellowish-white product and isolation, combination and evaporation of those fractions where, by means of the ninhydrin test, the presence of amino acids and/or peptides and/or protein-like substances is detectable.

**8.** Use according to claim 5, characterized in that a fraction obtained from the yellowish-white product is used as active substance, whereby the molecular weight of the fraction lies below 750, preferably in the region of 650-750.

**9.** Process for the isolation of new active-substance fractions of plant pollen extracts, in which the dried plant pollens are extracted by an extraction means containing water and a water-mixable organic solvent, potential high-molecular proteins are broken down completely or up to a maximum content of 5% by weight high-molecular proteins through the action of micro-organisms into low-molecular proteins or amino acids, the aqueous medium is separated from the pollen residues and is evaporated by vacuum, whereby the active-substance mixture remains in the form of a yellowish-white product, characterized in that from this active-substance mixture by means of gel electrophoresis a new active-substance fraction is isolated whose molecular weight lies below 750, whereby that fraction is preferably isolated whose molecular weight lies in the region of 650-750.

**10.** Active-substance fractions isolated from the plant pollen extracts, characterized in that these active-substance fractions are isolated in accordance with the process according to claim 9.

**Revendications**

**1.** Utilisation d'extraits de pollen pour la préparation de compositions pharmaceutiques, qui inhibent la croissance de tumeurs, caractérisée en ce qu'on utilise un extrait qui est obtenu à partir de pollen végétal par un agent d'extraction aqueux et qui contient au maximum 5% en poids de protéines de poids moléculaire élevé ou est exempt de celles-ci.

**2.** Utilisation suivant la revendication 1, caractérisée en ce qu'on utilise l'extrait pour la préparation d'une composition pharmaceutique qui inhibe la croissance de cellules cancéreuses après l'écoulement d'une phase de départ, ou qui inhibe la croissance de cellules de leucémie directement après le commencement du traitement et détruit éventuellement complètement celles-ci.

**3.** Utilisation suivant l'une des revendications 1 et 2, caractérisée en ce qu'on utilise un extrait sec qui présente une teneur en eau inférieure à 12%, par rapport au poids total de l'extrait, et qui contient au moins 70% en poids d'hydrates de carbone, 5 à 12% en poids d'acides aminés et/ou de peptides, ainsi que des vitamines du groupe B solubles dans l'eau.

**4.** Utilisation suivant l'une des revendications 1 et 2, caractérisée en ce que, pour la préparation de la composition pharmaceutique, on utilise une fraction de l'extrait de pollen qui est enrichie en acides aminés et/ou peptides et/ou substances analogues aux protéines.

5. Utilisation suivant l'une quelconque des revendications 1 à 4, selon laquelle, en vue de la préparation de compositions pharmaceutiques, on utilise un extrait de pollen qui a été obtenu par extraction de pollen végétal séché avec un agent d'extraction contenant de l'eau et un solvant organique miscible à l'eau et par la dégradation des protéines de poids moléculaire élevé éventuellement présentes par l'effet de microorganismes, où la dégradation des protéines de poids moléculaire élevé avec formation d'acides aminés ou de protéines de faible poids moléculaire, a été entreprise totalement ou jusqu'à une teneur maximale en protéines de poids moléculaire élevé de 5% en poids, par rapport au poids à sec, et où l'agent d'extraction contenant l'eau dont les résidus de pollen ont été séparés donne, après évaporation sous vide, le mélange de substances actives à utiliser sous forme d'un produit blanc jaunâtre.

6. Utilisation suivant la revendication 5, caractérisée en ce que l'on utilise, à titre de substance active, une fraction isolée du produit blanc jaunâtre, enrichie en acides aminés et/ou en peptides, où cette fraction fut préparée pour autant que l'on dissolvât le produit blanc jaunâtre dans de l'eau, on ajoutât un solvant organique miscible à l'eau et éventuellement un acide organique pour provoquer la précipitation des acides aminés, protéines et/ou substances analogues aux protéines et en ce qu'on utilise le précipité isolé après le séchage à titre de fraction à substance active.

7. Utilisation suivant la revendication 5, caractérisée en ce que l'on utilise, à titre de substance active, une fraction obtenue à partir du produit blanc jaunâtre, enrichie en acides aminés et/ou en peptides et/ou en substances analogues aux protéines, où l'on a obtenu la fraction par séparation par chromatographie sur colonne du produit blanc jaunâtre et isolement, purification et évaporation des fractions dans lesquelles on pouvait déceler la présence d'acides aminés et/ou de peptides et/ou de substances analogues aux protéines par un test à la ninhydrine.

8. Utilisation suivant la revendication 5, caractérisée en ce que l'on utilise, à titre de substance active, une fraction obtenue à partir du produit blanc jaunâtre, où le poids moléculaire de la fraction est inférieur à 750, de préférence compris dans la plage de 650 à 750.

9. Procédé d'isolement de nouvelles fractions à substance active d'extraits de pollen végétal, conformément auquel on extrait le pollen végétal séché à l'aide d'un agent d'extraction contenant de l'eau et un solvant organique miscible à l'eau, on dégrade les protéines de poids moléculaire élevé éventuellement présentes, totalement ou jusqu'à une teneur maximale de 5% en poids en protéines de poids moléculaire élevé, par l'effet de microorganismes, en acides aminés ou en protéines de faible poids moléculaire, on sépare le milieu aqueux des résidus de pollen et on l'évapore sous vide, de façon à laisser subsister le mélange à substance active sous forme d'un produit blanc jaunâtre, caractérisé en ce qu'à partir de ce mélange à substance active on isole, par électrophorèse sur gel, une nouvelle fraction à substance active dont le poids moléculaire est inférieur à 750, où l'on isole de préférence la fraction dont le poids moléculaire fluctue de 650 à 750.

10. Fractions à substance active isolées d'extraits de pollen végétal, caractérisé en ce que l'on a isolé ces fractions à substance active par mise en oeuvre du procédé selon la revendication 9.